# EUROPEAN PATENT SPECIFICATION

(11) **EP 0 471 040 B1**
(45) Date of publication and mention of the grant of the patent: **12.03.1997**
(21) Application number: 91900304.6
(22) Date of filing: 11.12.1990
(51) Int. Cl.: A61K 31/40

(54) **USE OF CROMAKALIM/BRL 38227 FOR THE TREATMENT OF HYPERREACTIVITY OF AIRWAYS**
VERWENDUNG VON CROMAKALIM/BRL 38227 ZUR BEHANDLUNG DER ÜBERREAKTIVITÄT DER LUFTWEGE
UTILISATION DE CROMAKALIM/BRL 38227 POUR LE TRAITEMENT DE L'HYPERREACTIVITE DES VOIES RESPIRATOIRES

(30) Priority: 11.12.1989 GB 8927980
(43) Date of publication of application: 19.02.1992
(73) Proprietor: Beecham Group p.l.c., Brentford, Middlesex TW8 9BD (GB)
(72) Inventor: BUCKLE, Derek Richard SmithKline Beecham, Bottom Road Epsom Surrey KT18 5XQ (GB); TAYLOR, John Frank SmithKline Beecham, Bottom Road Epsom Surrey KT18 5XQ (GB)
(74) Representative: Rutter, Keith, Dr.
(86) International application number: GB9001934
(87) International publication number: WO9108741

(56) References cited:
- Br. J. Pharmac., vol. 89, 1986, The Macmillan Press Ltd.; S.L. Allen et al.: "Electrical and mechanical effects of BRL34915 in guinea-pig isolated trachealis", pages 395-405
- Agents Actions Suppl., vol. 23, 1988, Birkhauser Verlag, Basel, CH; R.C. Small et al.: "K+-channel opening as a mechanism for relaxing airways smooth muscle", pages 89-94
- Br. J. Pharmacol., vol. 95, 1988, The Macmillan Press Ltd.; J.R.S. Arch et al.: "Evaluation of the potassium channel activator cromakalim (BRL 34915) as a bronchodilator in the guinea-pig: comparison with nifedipine", pages 763-770
- Br. J. Pharmacol. vol. 98, 1989, The Macmillan Press Ltd.; N.E. Bowring et al.: "Evaluation of inhaled cromakalim and its active enantiomer BRL 38277 as bronchodilators in the guinea-pig", page 805
- Japan J. Pharmacol. 63, pages 159-163 (1993)

## Description

The present invention relates to the use of a compound for the manufacture of a medicament for the treatment and/or prophylaxis of hyperreactivity of airways, associated with obstructive airways diseases, including asthma.

EP-A-176689 (Beecham Group p.l.c.) describes the use of benzopyran derivatives as bronchodilators.

It has now been discovered that in a model of peritoneal anaphylaxis, cromakalim/BRL 38227 inhibits extravasation and cellular infiltration, both of which are known components of the airways inflammatory response associated with obstructive airways disorders such as asthma. As such, these compounds are of value as disease modifying agents in the treatment or prophylaxis of these disorders. A particular aspect of the invention relates to the treatment or prophylaxis of airways hyper-responsiveness since this is believed to result from the underlying airways inflammation.

Accordingly, the present invention provides the use of a compound for the manufacture of a medicament for the treatment and/or prophylaxis of hyperreactivity of airways in mammals, such as humans, which use comprises administering to the mammal in need of such treatment and/or prophylaxis an effective and/or prophylactic amount of cromakalim or BRL 38227.

Cromakalim is the compound of Example 1 of EP-A-76075 and United States Patent No. 4446113, (±)-6-cyano-3,4-dihydro-2,2-dimethyl-trans-4-(2-oxo-1-pyrrolidinyl)-2H-benzo[b]pyran-3-ol, also known as cromakalim; and its (-)-enantiomer, also known as BRL 38227, disclosed in EP-A-120428.

Examples of pharmaceutically acceptable salts are as described in the aforementioned European Patent references, the subject matter of which are incorporated herein by reference.

References to cromakalim and BRL 38227, include solvates such as hydrates.

Cromakalim and BRL 38227 may be prepared as described in the aforementioned Patent Publications/References.

Preferably, cromakalim/BRL 38227 is in substantially pure pharmaceutically acceptable form.

The administration of the compound may be by way of oral, sublingual, transdermal or parenteral administration. A composition may also be in the form of a spray, aerosol or other conventional method of inhalation.

An amount effective to treat the disorders hereinbefore described depends on the usual factors such as the nature and severity of the disorders being treated and the weight of the mammal. However, a unit dose will normally contain 0.1 to 50 mg for example 0.5 to 10 mg, of the potassium channel activator, such as pinacidil or the compound of formula (I) or a pharmaceutically acceptable salt thereof. Unit doses will normally be administered once or more than once a day, for example 2, 3, or 4 times a day, more usually 1 to 3 times a day, such that the total daily dose is normally in the range, for a 70 kg adult of 0.1 to 50 mg, for example 0.5 to 10 mg, that is in the range of approximately 0.001 to 1 mg/kg/day, more usually 0.005 to 0.2 mg/kg/day.

For oral or parenteral administration, it is greatly preferred that the potassium channel activator is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling or tabletting. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing the potassium channel activator and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound.

For transdermal administration, formulations suitable for topical use may be employed, optionally containing penetration enhancers.

As is common practice, the compositions will usually be accompanied by written or printed directions for use in the treatment concerned.

The present invention also provides the use cromakalim/BRL 38227 in the manufacture of a medicament for use in the treatment and/or prophylaxis of hyperreactivity of airways. Such treatment and/or prophylaxis may be carried out as hereinbefore described.

The present invention further provides a pharmaceutical composition for use in the treatment and/or prophylaxis of hyperreactivity of airways which comprises cromakalim/BRL 38227 or a pharmaceutically acceptable salt thereof; and a pharmaceutically acceptable carrier.

Such compositions may be prepared in the manner as hereinbefore described.

Cromakalim/BRL 38227 is a potassium channel activator, and the present invention may also be extended to other compounds which are potassium channel activators, such as those described in EP-A-372998 (Beecham Group p.l.c.), EP-A-360621 (Ortho Pharmaceutical Corp.), EP-A-365416 (Adir and Co), EP-A-344747 (Fujisawa), EP-A-350805 (Beiersdorf), EP-A-326297 (Rhone-Poulenc), U.K. Patent No. 1489879 (E. Lilly) (in particular Examples 1 and 47), EP-A-355565 (Hoechst Aktiengesellschaft), EP-A-363883 (Merck Patent GmbH) and EP-A-354553 (E.R. Squibb and Sons Inc.)

### PHARMACOLOGICAL DATA

### Materials and Methods

Animals were sensitised according to the procedure of Spicer et al. Agents and Actions 17, 498-505 (1985). Male rats were sensitised by the intradermal injection of 100 µg bovine serum albumin (BSA) in 0.2 ml of Freunds complete adjuvant and 28 days later given a booster injection of a further 100 µg of BSA in 0.1 ml of isotonic saline by subcutaneous injection. On day 34 the rats were given a suspension of 0.5 mg of Sephadex® G200 particles in 1 ml of isotonic saline by intravenous injection to induce a blood eosinophilia. Six days later the rats were challenged by the intraperitoneal injection of 0.5 mg BSA in 0.5 ml of isotonic saline. Immediately before challenge, rats were given 0.5 ml of 5% solution of Pontamine Sky Blue dye in isotonic saline intravenously to label serum proteins.

Four hours after challenge, the peritoneal cavities were washed out with 5 ml of saline containing 6 units of heparin/ml. Negative control rats were treated similarly except that BSA was omitted at the time of sensitisation and boost. 20µl samples of the peritoneal washings were added to 10ml of Isoton 11 and Zaponin (2 drops) was added within 30 min, to lyse erythrocytes, just prior to total cell count determination using a Coulter counter. Differential cell counts were determined as previously described (Spicer et al, loc.cit), a total of 400 leucocytes being counted.

The concentration of blue dye in the supernatants was determined by spectromorphic analysis at 625 nm, after centrifugation to remove cellular material. This gave a measure of the amount of dye labelled plasma proteins extravasated into the peritoneal cavity in the 4 hour period from challenge until the fluid was collected, although most of the extravasation occurred within the first 10 min after challenge.

The biological results of BRL 38227 (3S,4R-6-cyano-3,4-dihydro-2,2-dimethyl-4-(2-oxopyrrolidin-1-yl)-2H-1-benzopyran-3-ol) are shown in the table.

### Effects of oral BRL 38227 on extravasation and cellular infiltration in the peritoneal cavity of actively sensitized rats

Test compound or vehicle were given 30 min before challenge; pontamine sky blue was given i.v. immediately before challenge to assess extravasation. Sham sensitized animals received vehicles, except for the pontamine sky blue and the BSA challenge on day 40. Peritoneal washings were collected four hours after antigen challenge.
n=6 or 7. Results are means±S.E. *P<0.05; **P*<0.01; ***P<0.001 compared to controls.

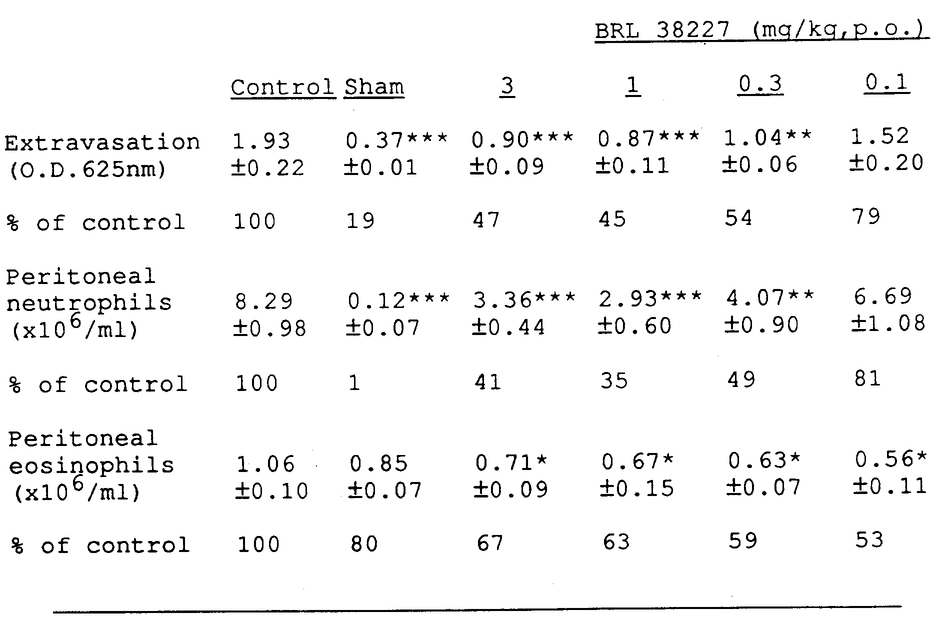

## Claims

1. Use of a potassium channel activator for the manufacture of a medicament for use in the treatment or prophylaxis of hyperreactivity of airways.

2. A use according to claim 1, wherein the potassium channel activator is (±)-6-cyano-3,4-dihydro-2,2-dimethyl*-trans-*4-(2-oxo-1-pyrrolidinyl)-2H-benzo[b]pyran-3-ol, and its (-)-enantiomer.

## Patentansprüche

1. Verwendung eines Kalium-Kanal-Aktivators zur Herstellung eines Arzneimittels zur Verwendung bei der Behandlung oder Vorbeugung von Hyperreaktivität der Atemwege.

2. Verwendung gemäß Anspruch 1, wobei der Kalium-Kanal-Aktivator (±)-6-Cyano-3,4-dihydro-2,2-dimethyl-trans-4-(2-oxo-1-pyrrolidinyl)-2H-benzo[b]pyran-3-ol oder sein (-)-Enantiomer ist.

## Revendications

1. Utilisation d'un activateur du passage du potassium, pour la fabrication d'un médicament en vue de l'emploi dans le traitement ou la prophylaxie de l'hyper-activité des voies respiratoires.

2. Utilisation selon la revendication 1, dans laquelle l'activateur du passage du potassium est le(±)-6-cyano-3,4-dihydro-2,2-diméthyl-trans-4-(2-oxo-1-pyrrolidinyl)-2H-benzo|b|pyran-3-ol, et son énantiomère (-).
